(19) Europäisches Patentamt
European Patent Office
Office européen des brevets

(11) Numéro de publication: **0 365 416 B1**

(12) **FASCICULE DE BREVET EUROPEEN**

(45) Date de publication de fascicule du brevet: **15.09.93**

(51) Int. Cl.$^5$: **C07D 405/04**, A61K 31/40

(21) Numéro de dépôt: **89402857.0**

(22) Date de dépôt: **17.10.89**

Le dossier contient des informations techniques présentées postérieurement au dépôt de la demande et ne figurant pas dans le présent fascicule.

# (54) Nouveaux dérivés de l'amino chromanol, leur procédé de préparation et les compositions pharmaceutiques les renfermant.

(30) Priorité: **17.10.88 FR 8813637**

(43) Date de publication de la demande:
**25.04.90 Bulletin 90/17**

(45) Mention de la délivrance du brevet:
**15.09.93 Bulletin 93/37**

(84) Etats contractants désignés:
**AT BE CH DE ES FR GB GR IT LI LU NL SE**

(56) Documents cités:
**EP-A- 0 277 611**

**JOURNAL OF MEDICINAL CHEMISTRY, vol. 29, 1986, pages 2194-2201, American Chemical Society, Washington, DC, US; V.A. ASHWOOD et al.: "Synthesis and antihypertensive activity of 4-(cyclic amido)-2H-1-benzopyrans"**

(73) Titulaire: **ADIR ET COMPAGNIE**
**1 rue Carle Hébert**
**F-92415 Courbevoie Cédex(FR)**

(72) Inventeur: **Régnier, Gilbert**
**Demeure du Plessis - Bât. D 27 Avenue du Plessis**
**F-92290 Chatenay Malabry(FR)**
Inventeur: **Dhainaut, Alain**
**7 rue des Guipières**
**F-78400 Chatou(FR)**
Inventeur: **Vilaine, Jean-Paul**
**3 avenue des Fusillés**
**F-92290 Chatenay Malabry(FR)**
Inventeur: **Villeneuve, Nicole**
**135 rue Danton-Bât. A1**
**F-92500 Rueil Malmaison(FR)**
Inventeur: **Joly, Ghislaine**
**28 rue des Poissonniers**
**F-92200 Neuilly s/Seine(FR)**
Inventeur: **Duhault, Jacques**
**14bis rue Paul Demange**
**F-78290 Croissy s/Seine(FR)**

(74) Mandataire: **Reverbori, Marcelle**
**Adir et Compagnie, 1, rue Carle Hébert**
**F-92415 Courbevoie Cédex (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 365 416 B1

## Description

La présente invention a pour objet de nouveaux dérivés de l'amino chromanol, leur procédé de préparation et les compositions pharmaceutiques les renfermant.

Elle concerne particulièrement les dérivés de l'amino chromanol de formule générale I :

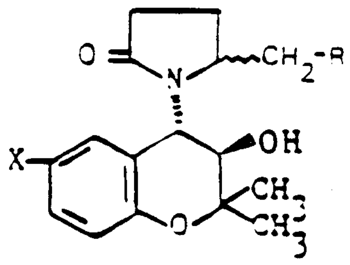

et

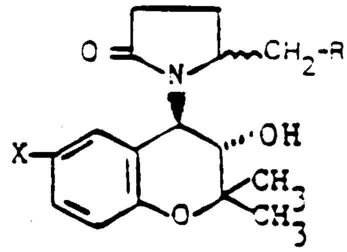

(I)

dans laquelle :

. X représente un radical cyano, nitro, trifluorométhyle, alkoxycarbonyle dans lequel le groupe alkoxy renferme de 1 à 5 atomes de carbone en chaîne droite ou ramifiée, un radical carbamido, dialkylcarbamido, aminosulfonyle, dialkylaminosulfonyle, alkylsulfonyle ou alkylsulfonylamino dans lesquels le groupe alkyle renferme de 1 à 5 atomes de carbone, ou un radical acyle tel que par exemple un radical acétyle, propionyle, benzoyle ou trifluoroacétyle ;

. $CH_2$-R est fixé (en position 5 du noyau pyrrolidinone) sur un atome de carbone de configuration R ou S, et

. R représente :

a) soit un radical -OR' ou SR' dans lequel R' représente : un atome d'hydrogène ;

un radical alkyle jusqu'en $C_{10}$, en chaîne droite ou ramifiée, comportant éventuellement un ou plusieurs atomes d'oxygène et/ou un radical hydroxy ;

un radical alkylcarbonyle dans lequel le groupe alkyle renferme jusqu'à 5 atomes de carbone en chaîne droite ou ramifiée ;

un reste

$$(CH_2)n-N < {R_1 \atop R_2}$$

dans lequel

n est un nombre entier de 2 à 5

et $R_1$ , $R_2$ identiques ou différents représentent chacun :

. un atome d'hydrogène,

. un radicale alkyle ayant jusqu'à 5 atomes de carbone en chaîne droite ou ramifiée, ou

. $R_1$ et $R_2$ forment ensemble avec l'atome d'azote auquel ils sont liés un cycle ayant 4 ou 5 atomes de carbone, comportant éventuellement un autre hétéroatome tel qu'un atome d'oxygène ou de soufre, ou un groupe -N-$R_3$ dans lequel $R_3$ représente

. un atome d'hydrogène,

. un radical alkyle ayant jusqu'à 5 atomes de carbone en chaîne droite ou ramifiée, ou

. un reste -AZ dans lequel

A représente une liaison simple ou une chaîne hydrocarbonée de $C_1$ à $C_5$ comportant éventuellement une double liaison, et

Z représente

. un radical hydroxy,

. un radical benzhydryle,

. un radical phényle éventuellement substitué par un ou plusieurs atomes d'halogène, ou radicaux trifluorométhyle, alkoxy de $C_1$ à $C_5$ ou méthylènedioxy, ou

. un radical hétérocyclique penta ou hexagonal comportant un ou plusieurs atomes d'oxygène, d'azote ou de soufre tels que les radicaux pyridyle, thiazolyle, pyrimidinyle, ou pyrazinyle, chacun éventuellement substitué par un ou plusieurs atomes d'halogène ou radicaux alkyle ou alkoxy chacun de $C_1$ à $C_5$ ;

b) soit un radical

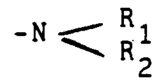

dans lequel $R_1$ et $R_2$ ont les significations énoncées précédemment.

L'état antérieur de la technique le plus proche de la présente invention est illustré notamment par :

- J.M. EVANS et coll. J. med. chem. 29 (II) 2194-2201 (1986) ;
- la demande de brevet européen publiée sous le n° 0076075, le 06.04.1983 qui concerne des composés du benzopyranne parmi lesquels le produit leader est le CROMAKALIM répondant à la formule :

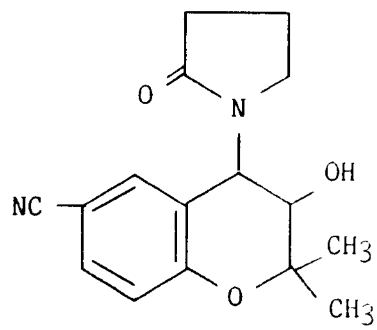

laquelle formule ne contient aucun substituant sur le noyau pyrrolidone, et

- la demande de brevet européen EP-A-277611 qui a, entre autres, pour objet les dérivés de formule :

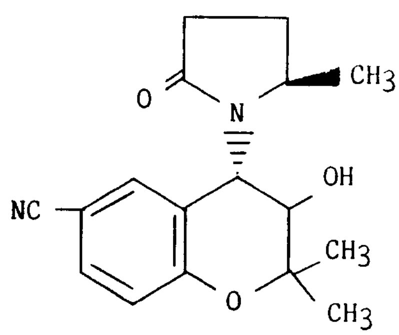

Il a été trouvé dans les services de la demanderesse que l'introduction d'un substituant $CH_2$-R, R étant tel que précédemment défini, en position a de l'atome d'azote du noyau pyrrolidone conduit aux dérivés I de la présente invention lesquels dérivés sont des diastéréoisomères purs qui présentent, par rapport au dérivé racémique qu'est le CROMAKALIM un avantage particulièrement intéressant sur le plan de l'application thérapeutique.

En effet, les dérivés de la présente invention -dont le profil pharmacologique est celui d'agoniste potassique ayant une application notamment dans le domaine cardiovasculaire- ont, selon les dérivés, une activité physiologique comparable ou supérieure à celle du CROMAKALIM avec une durée d'action beaucoup plus d'où leur avantage majeur pour leur utilisation en thérapeutique.

La présente invention a également pour objet le procédé de préparation des dérivés de formule générale I, selon le schéma réactionnel A :

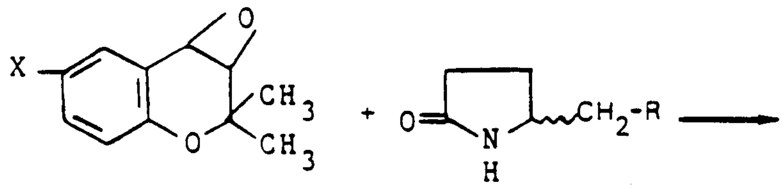 (I)

(II) (III)

caractérisé en ce que l'on fait réagir, en quantité équimoléculaire le dérivé du chromane de formule générale II, dans laquelle X a la signification précédemment définie, sur le dérivé chiral, de configuration R ou S, de la pyrrolidone de formule générale III dans laquelle R a la signification précédemment définie à l'état de sel de sodium ou de lithium.

Il est particulièrement adéquat d'effectuer la réaction dans un solvant aprotique, à une température comprise entre 20 et 50 ° C.

Comme solvants aprotiques particulièrement appropriés ont peut citer par exemple le diméthylsulfoxyde, l'hexaméthylphosphorotriamide, le diméthylformamide et le diméthylacétamide.

La pyrrolidone III doit être transformée préalablement en sel de sodium ou de lithium au moyen d'hydrure de sodium ou de butyl lithium, dans le solvant choisi pour effectuer la réaction.

Les dérivés du chromane II peuvent être préparés selon la technique de J.M. EVANS et coll., J. med. chem. 26 (II) 1582-89 (1983) et E.P. 0076.075.

Les dérivés chiraux de configuration R ou S, de la pyrrolidone III ont été préparés respectivement à partir des acides pyroglutamiques naturels de configuration R ou S, selon le schéma B suivant :

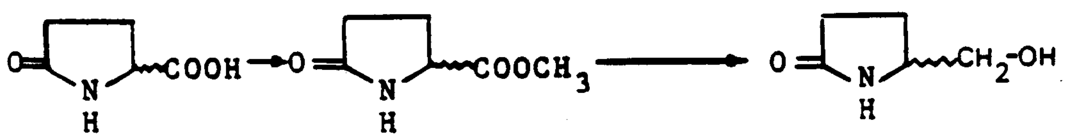

(IV) (V) (VI)

décrit par S. SAIJO et coll., Chem. Pharm. Bull. 28, 1449 ( 1980).

Le pyroglutaminol R ou S VI peut servir de matière première pour la préparation des pyrrolidones chirales III

- soit directement, das le cas où R = OR', selon le schéma C :

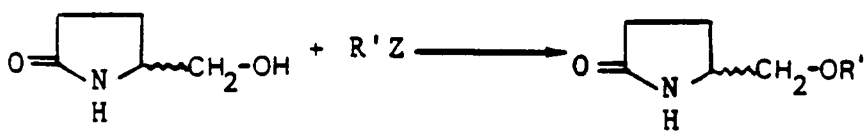

(VI) (III)

Z étant un atome d'halogène tel que brome ou iode, un ion sulfate ou arylsulfonate, et R' ayant la signification précédemment définie ;
la réaction étant généralement effectuée dans l'eau ou un solvant aprotique polaire en présence d'une base forte ou d'une amine tertiaire faisant office d'accepteur de l'acide formé ;

- soit, après transformation par des méthodes connues en dérivé halogéné, de préférence chloré de formule:

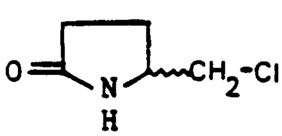 (VII)

que l'on fait réagir selon le schéma D :

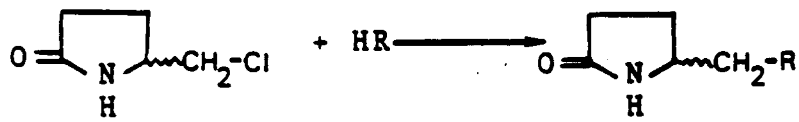

(VII) (VIII)

R étant soit -OR' ou SR' soit

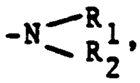

la réaction étant généralement effectuée selon des méthodes connues dans un solvant aprotique polaire, à partir d'un dérivé alcalin du composé R′OH ou en présence d'un excès d'amine

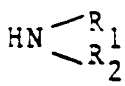

ou d'une base tertiaire faisant office d'accepteur de l'hydracide formé.

Dans le cas particulier des dérivés de formule I dans laquelle R représente OR′ et R′ un atome d'hydrogène, c'est-à-dire pour préparer les dérivés de formule I' :

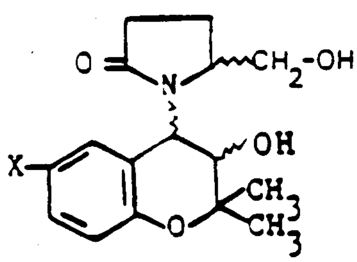

(I')

il est préférable d'effectuer la réaction selon le schéma A donné précédemment, à partir d'une pyrrolidone chirale, de configuration R ou S, de formule

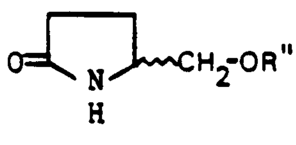

dans laquelle R" est un reste labile facilement hydrolysable,
que l'on peut préparer à partir du dérivé VI et de l'éthyl vinyl éther, selon une technique décrite par S. SAIJO et coll. Chem. Pharm. Bull. 28, 1449 (1980).

Les dérivés de formule générale I sont des substances chirales diastéréoisomères.
La méthode de synthèse selon le schéma A fournit toujours des amino chromanols I dans la configuration trans par ouverture de la fonction époxyde, et l'emploi de pyrrolidone III de configuration S ou R fournira, (après séparation par chromatographie sur colonne ou par formation de sels et cristallisation de ceux-ci dans le cas où R contient une fonction basique) deux paires de diastéréoisomères purs de configuration 3S,4R,5'S et 3R,4S,5'S ou 3S,4R,5'R et 3R,4S,5'R.

La séparation sur colonne s'effectue par chromatographie flash, sous des pressions d'azote comprises entre 0,5 et 1 bar, avec de la silice de 35-70 μ et des systèmes de solvants tels que : $CH_2Cl_2$- méthanol ou $CH_2Cl_2$- acétate d'éthyle.

La formation de sels peut s'effectuer à partir des composés I dans lesquels R contient une fonction basique, et d'acides minéraux ou organiques et recristallisation de ces sels dans un solvant approprié. Ces sels sont également inclus dans la présente invention.

Les dérivés de formule générale I et leurs sels physiologiquement tolérables possèdent des propriétés pharmacologiques et thérapeutiques intéressantes, notamment des propriétés modulatrices du potentiel de membrane cellulaire.

Ces substances activatrices de canaux potassiques membranaires augmentent un courant potassique sortant et induisent ainsi une hyperpolarisation membranaire.

Le mécanisme d'action de cette nouvelle classe pharmacologique permet d'aborder de façon originale les anomalies de la régulation du potentiel de membrane cellulaire et de la régulation ionique cellulaire, incluant la modulation des mouvements calciques transmembranaires et intra cellulaires, outre celle des flux potassiques.
Ces anomalies sont impliquées dans de nombreux processus physiopathologiques associés notamment à des troubles de régulation de la contraction des différents types de muscles lisses.

Les essais pharmacologiques réalisés in vitro montrent que ces produits ont une puissante activité relaxante vasculaire compatible avec une activation de canaux potassiques membranaires.

Testés in vivo sur des modèles d'hypertension artérielle chez le rat et le chien ces substances administrées par voie orale ont une puissante activité antihypertensive d'une durée très nettement supérieure à celle de tous les composés, actuellement décrits, ayant un tel mécanisme d'action en particulier le CROMAKALIM, produit le plus proche de l'Art antérieur.

Les études hémodynamiques effectuées chez le chien indiquent la puissance et la longue durée de leur effet vasodilatateur périphérique et coronaire.

Ces propriétés permettent l'utilisation des dérivés de l'invention comme médicament, notamment dans le domaine cardiovasculaire : l'hypertension artérielle systémique et pulmonaire, l'ischémie myocardiaque, les maladies vasculaires périphériques et l'insuffisance cardiaque et plus généralement le traitement et la prévention des troubles liés au vieillissement artériel et à l'athérosclérose. Ils peuvent être également utilisés dans les pathologies métaboliques constituant un facteur de risque cardiovasculaire tels : l'obésité, le diabète et les dyslipidémies.

De plus, l'activité de ces composés sur les différents types de muscles lisses permet d'élargir leur cadre thérapeutique aux affections associées à des troubles de régulation de la contraction musculaire lisse dans la sphère bronchopulmonaire (bronchopneumopathie obstructive, asthme) digestive, urinaire et utérine.

Ces indications thérapeutiques ne sont pas limitatives dans la mesure où l'altération du potentiel de membrane cellulaire quelqu'en soit la cause et la localisation tissulaire aboutit à une disfonction cellulaire, source de phénomènes pathologiques, et peut constituer une cible thérapeutique majeure des produits décrits.

Ainsi de nombreuses situations de souffrance tissulaire qu'elles soient liées au vieillissement, à l'ischémie, l'inflammation ou l'oedème (en particulier au niveau cérébral) peuvent être traitées ou prévenues par les produits de la présente invention.

La présente invention a également pour objet les compositions pharmaceutiques contenant comme principe actif un composé de formule générale I ou un de ses sels physiologiquement tolérable, mélangé ou associé à un excipient pharmaceutique approprié, comme par exemple, l'eau distillée, l'amidon, le talc, l'éthylcellulose ou le stéarate de magnésium.

Les compositions pharmaceutiques ainsi obtenues se présentent généralement sous forme dosée et peuvent contenir de 0,05 à 50mg de principe actif. Elles peuvent revêtir par exemple, la forme de comprimés, dragées, gélules, suppositoires, solutions injectables ou buvables ainsi que les formes adaptées à l'administration par aérosol, et être selon les cas administrées par voie orale, rectale ou parentérale à la dose de 0,05 à 50mg, 1 à 2 fois par jour.

Les exemples suivants illustrent l'invention.

**Exemple 1 :**

3R, 4S ou 3S, 4R trans diméthyl-2,2 hydroxy-3 (oxo-2 hydroxyméthyl-5S pyrrolidinyl-1)-4 cyano-6 chromane, (formes A et B)

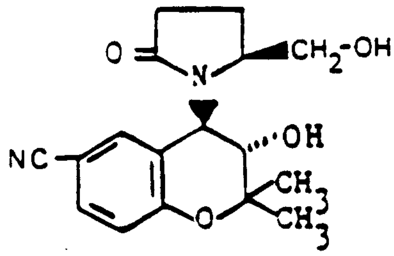

et

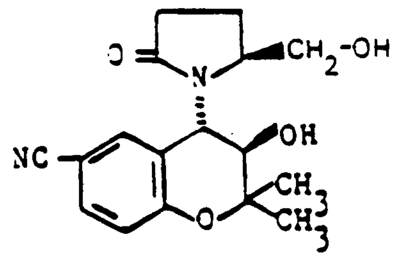

A une solution de 10,5g de S(éthoxy-1 éthoxy méthyl-5) pyrrolidone-2 dans 20ml d'hexaméthyl phosphorotriamide, on ajoute 39ml d'une solution de butyl lithium 1,6M dans l'hexane, à 15-20 °C. On agite 30 minutes à 20 °C puis ajoute 14,1g de cyano-6 diméthyl-2,2 époxy-3,4 chromane, fondant (capillaire) à 97-98 °C. On chauffe 20 heures à 35 °C, refroidit et ajoute 3,7ml d'acide acétique en agitant 30 minutes à 5-10 °C. On dilue avec 200ml d'eau, et agite encore pendant 30 minutes à 5-10 °C. Un produit huileux se sépare. On décante le jus aqueux, reprend l'huile de nouveau avec 150ml d'eau. Il se forme alors un précipité. On ajoute du $CH_2Cl_2$, agite et décante la couche organique. Après évaporation on obtient 23g d'huile brute que l'on dissout dans 100ml de méthanol. On agite la solution avec 50ml d'HCL 0,1N pendant 2 heures pour éliminer le groupement protecteur. Après évaporation du solvant ou chromatographie sur colonne flash à travers 1kg de silice et le système $CH_2Cl_2$- méthanol 95-5 comme éluant, on isole deux fractions pures de Rf = 0,46 et 0,49 correspondant aux deux diastétéoisomères purs :
forme A : 4,6g P.F. (cap) = 152-153 °C,

$$[\alpha]_D^{20°5} = -32°7 \text{ (C=1 EtOH)}$$

forme B : 3,8g P.F. (cap) = 156-158 °C

$$[\alpha]_D^{20°5} = -62°9 \text{ (C=1 EtOH)}$$

La S(éthoxy-1 éthoxy méthyl-5) pyrrolidone-2 de départ, (huile)

$$[\alpha]_D^{20°5} = +21°8$$

(C = 1 EtOH), a été préparée, avec un rendement de 96%, par addition d'éthyl vinyl éther dans $CHCl_3$, en présence de $CF_3COOH$, à la S hydroxyméthyl-5 pyrrolidone-2, (huile) $[\alpha]_D^{21}$ = + 33 ° 5 (C = 3 EtOH)

**Exemple 2 :**

3S,4R ou 3R, 4S trans-diméthyl-2,2 hydroxy-3 (oxo-2 méthoxyméthyl-5S pyrrolidinyl-1)-4 cyano-6 chromane (formes A et B)

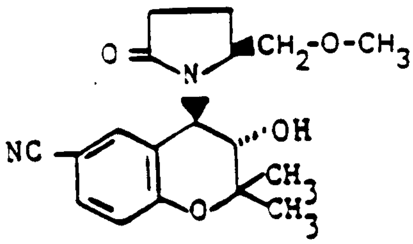

et

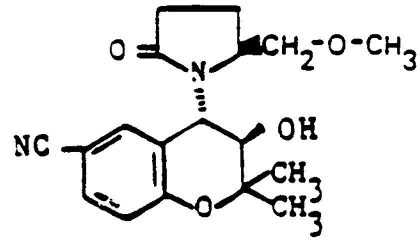

Les formes :
A , $[\alpha]_D^{21}$ = -69°6 (C = 1 EtOH), point de fusion capillaire 186-189 °C
B , $[\alpha]_D^{21}$ = +1°22 (C = 1 EtOH),point de fusion capillaire 142-145 °C
ont été préparées selon la méthode décrite dans l'exemple 1, à partir de la S méthoxyméthyl-5 pyrrolidone-2 (huile), elle-même préparée à partir de la S hydroxyméthyl-5 pyrrolidone-2 et du diméthylsulfate en présence de soude, avec un rendement de 60%.

**Exemple 3 :**

3R, 4S ou 3S, 4R trans diméthyl-2,2 hydroxy-3 (oxo-2 diméthylaminométhyl-5S pyrrolidinyl-1)-4 cyano-6 chromane, (formes A et B ) :

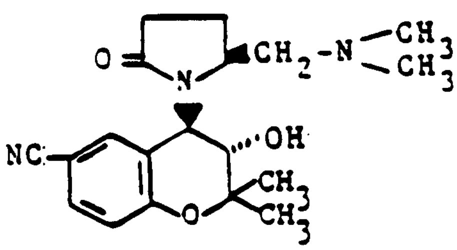

et

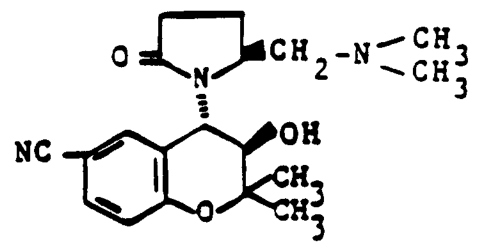

Les formes :
A , P.F. (cap) = 153-156 °C,

$$[\alpha]_D^{20°5} = -39°$$

(C = 1 EtOH)
B oxalate, P.F. (cap) = 194-197 °C,

$$[\alpha]_D^{20°5} = -41°1$$

(C-1 DMSO)
ont été préparées, selon la méthode décrite dans l'exemple 1, à partir de la S diméthylaminométhyl-5 pyrrolidone -2 (huile) à 96% de pureté optique (HP LC), elle-même préparée par chauffage à 100 °C avec un excès de diméthylamine dans l'éthanol, de la S chlorométhyl-5 pyrrolidone-2 (huile) à 94% de pureté optique (HP LC, colonne chirale).

**Exemples 4-30 :**

En opérant comme dans l'exemple 1, à partir des matières premières appropriées, ont été préparés les produits objet des exemples suivants (sous formes A et B) :

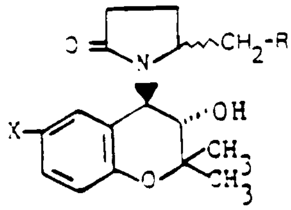

et

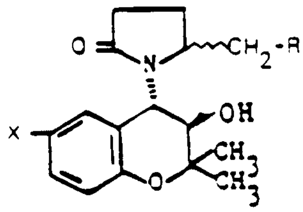

| n° Ex | X | R |
|---|---|---|
| 4 | -CN | $-O-CO-CH_3$ |
| 5 | -CN | $-O-(CH_2)_5-CH_3$ |
| 6 | -CN | $-O-CH_2CH_2-O-CH_3$ |
| 7 | -CN | $-O-(CH_2)_2-N(-CH_3)_2$ |
| 8 | -CN | $-O(CH_2)_2-N(-C_2H_5)_2$ |
| 9 | -CN | $-N(-C_2H_5)_2$ |
| 10 | -CN | 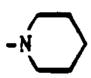 |
| 11 | -CN | 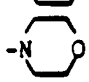 |
| 12 | -CN | 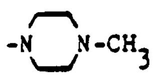 |
| 13 | -CN | 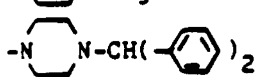 |
| 14 | -CN | 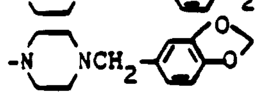 |
| 15 | -CN | 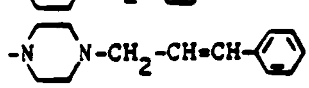 |
| 16 | -CN | 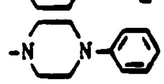 |
| 17 | -CN | 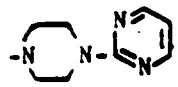 |
| 18 | -CN | 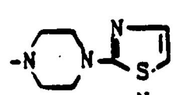 |
| 19 | -CN | 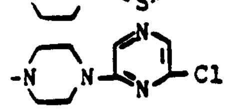 |

| n° Ex. | X | R |
|---|---|---|
| 20 | -CN | 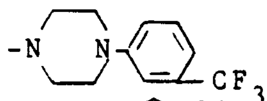 |
| 21 | -CN | 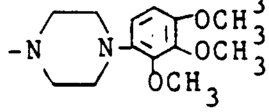 |
| 22 | -CN | $-O-CH_2-CH_2-OH$ |
| 23 | $-NO_2$ | -OH |
| 24 | $-\underset{\overset{\|}{O}}{C}-OCH_3$ | -OH |
| 25 | -CN | -SH |
| 26 | $-CO-CH_3$ | -OH |
| 27 | $-SO_2CH_3$ | -OH |
| 28 | $-SO_2N\begin{matrix}CH_3\\CH_3\end{matrix}$ | -OH |
| 29 | $-CF_3$ | -OH |
| 30 | $-NH-O_2S-CH_3$ | -OH |

**Exemple 20 :**

Etude pharmacologique in vitro,dans le domaine cardiovasculaire :

Des rats mâles wistar (325-375g) sont anesthésiés par voie I.P. au pentobarbital sodique (30mg/kg). L'aorte thoracique et la veine porte mésentérique sont prélevées. L'aorte est disséquée en anneaux de 3mm de longueur et l'endothélium est éliminé mécaniquement.
La veine porte est dégagée sur 1cm de longueur et attachée à ses extrémités.
Les organes sont placés dans une cuve thermostatée de 20ml contenant une solution physiologique composée de (mM) : NaCl 112 ; KCl 5 ; $KH_2PO_4$ 1 ; Mg $SO_4$ 1,2 ; Ca $Cl_2$ 2,5 ; Na $HCO_3$ 25 ; glucose 11,5 ; EDTA 0,1 ; pH : 7,4; 37 ° C ; 95% $O_2$ + 5% $CO_2$.

La tension initiale appliquée est de 2g pour l'aorte et de 0,5g pour la veine porte.
Les variations de tension sont mesurées par un capteur STATHAM connecté à un amplificateur transducer (GOULD) et suivies en continu sur un enregistreur graphique GOULD 2400.

Les protocoles expérimentaux sont les suivants :
Après une période de stabilisation de 90 minutes, les anneaux aortiques sont soumis à différents tests :

- dépolarisation potassique :
  Les préparations sont soumises à un milieu hyperpotassique 30mM ou 80mM de KCl (et respectivement 87 et 37mM de Na Cl) ; après 30 minutes de stabilisation de la contraction, des concentrations cumulatives de produit à tester sont additionnées toutes les 15 minutes.
  Les valeurs de relaxation obtenues permettent le calcul des $IC_{50}$.

- réactivité à la 4-aminopyridine (4 AP)
  Le produit à tester, à une concentration donnée, ou son solvant, est mis en incubation dans la solution physiologique normale 15 minutes avant l'addition de la 4 AP (5mM).
  Les amplitudes maximum des réponses à la 4 AP en présence de produit ou de solvant sont comparées.
  . La veine porte présente une activité rythmique spontanée stable en 60 minutes. Des concentrations cumulatives de produit à tester sont additionnées au milieu toutes les 15 minutes ; les variations de fréquence et d'amplititude des pics sont enregistrées.
  . Les produits sont utilisés sous forme dissoute dans l'eau ou le solvant approprié.
  . Les concentrations exprimées font référence aux concentrations finales en terme de base dans les bains d'organes isolés.
  . Les résultats obtenus sont regroupés dans le tableau suivant :

| Produits testés | AORTE DE RAT | | | VEINE PORTE |
|---|---|---|---|---|
| | IC50(M) KCl | | %d'inhibition de la réponse à la 4AP selon la concentration de produit (M) | Suppression de l'activité rythmique(M) |
| | 30mM | 80mM | | |
| **CROMAKALIM** | $4.210^{-7}$ | $> 10^{-4}$ | 13% ($10^{-7}$)<br>74% ($10^{-5}$) | $3\ 10^{-7}$ |
| Ex. 1 Forme A | $7.710^{-6}$ | $> 10^{-4}$ | 35% ($10^{-5}$) | $10^{-5}$ |
| Ex. 1 Forme B | $3.410^{-7}$ | $> 10^{-4}$ | 18% ($3.10^{-6}$)<br>77% ($10^{-5}$) | $310^{-7}$<br>$10^{-5}$ |
| Ex. 2 Forme A | $2.810^{-7}$ | $> 10^{-4}$ | | |

L'examen de ce tableau montre que :

. Les produits testés présentent une puissante activité relaxante vasculaire en milieu dépolarisé par une faible concentration potassique (30mM) disparaissant à forte concentration potassique (80mM).

. Les contractions vasculaires induites par la 4 aminopyridine (antagoniste de canaux potassiques) sont puissamment inhibées par ces composés.

. L'activité rythmique spontanée de la veine porte sensible aux variation du potentiel de membrane est profondément altérée par les produits décrits.

. L'ensemble de ces résultats est compatible avec un mécanisme d'action hyperpolarisant membranaire par activation de canaux potassiques.

. La puissance d'activité du produit de l'exemple 1 forme B est comparable sur ces tests à celle du plus puissant composé de référence connu : le CROMAKALIM

**Exemple 21 :**

Etude pharmacologique in vivo,dans le domaine cardiovasculaire :
A- Elle comprend les tests suivants :

1- ETUDE CHEZ LE RAT SPONTANEMENT HYPERTENDU (SHR)

1-1- METHODOLOGIE

L'étude est réalisée sur des rats mâles SHR (Charles River) pesant entre 260g et 330g, âgés de 18 à 20 semaines. Un cathéter en polyéthylène est mis en place au niveau de l'aorte abdominale et tunellisé au niveau du cou sous anesthésie (chlorhydrate de kétamine 150mg/kg par voie intra péritonéale). Les animaux, placés en cage de contention, sont testés à l'état éveillé au moins 48 heures après l'intervention. La pression artérielle, mesurée par un capteur de pression STATHAM P23 relié au cathéter abdominal, est recueillie sur un enregistreur GOULD 2400.
Les produits sont administrés par voie orale sous forme dissoute (eau ou solvant approprié) sous un volume

de 2,5mg/kg. Les doses administrées sont exprimées en mg/kg de base.

1-2- RESULTATS

Les résultats obtenus avec un certain nombre de produits représentatifs de l'invention et un produit de référence le CROMAKALIM sont regroupés dans le tableau suivant. Les variations de pression artérielle systolique (PAS) et diastolique (PAD), présentées au temps les plus significatifs après traitement, sont exprimées en pourcentage de variation par rapport aux valeurs contrôles.

| Produits testés | DOSE mg/kg | N | VALEURS CONTROLE mmHg | VARIATIONS ( %) EN FONCTION DU TEMPS APRES TRAITEMENT | | | |
|---|---|---|---|---|---|---|---|
| | | | | 30 mn | 1 H | 3 H | 6 H |
| CROMAKALIM | 0.3 | 6 | PAS 202 + -9<br>PAD 129 + -6 | -29 + -2<br>-34 + -2 | -21 + -3<br>-26 + -3 | -5 + -4<br>-7 + -5 | -1 + -2<br>-3 + -1 |
| CROMAKALIM | 1.0 | 6 | PAS 211 + -10<br>PAD 136 + -8 | -34 + -5<br>-44 + -4 | -23 + -4<br>-33 + -3 | -19 + -3<br>-25 + -4 | -15 + -2<br>-20 + -2 |
| Exemple 1 forme B | 0.3 | 4 | PAS 219 + -7<br>PAD 146 + -5 | -27 + -5<br>-34 + -6 | -27 + -4<br>-36 + -4 | -25 + -3<br>-35 + -4 | -29 + -3<br>-40 + -4 |
| Exemple 1 forme B | 1.0 | 4 | PAS 213 + -5<br>PAD 135 + -5 | -42 + -2<br>-48 + -5 | -44 + -1<br>-54 + -2 | -40 + -2<br>-46 + -6 | -36 + -3<br>-46 + -4 |
| Exemple 2 forme A | 1.0 | 5 | PAS 192 + -4 PAD 118 + -3 | -11 + -2<br>-17 + -3 | -16 + -3<br>-21 + -4 | -12 + -3<br>-19 + -3 | -9 + -2<br>-14 + -2 |
| Exemple 2 forme A | 3.0 | 6 | PAS 203 + -3<br>PAD 130 + -3 | -24 + -1<br>-35 + -3 | -26 + -2<br>-36 + -3 | -25 + -3<br>-31 + -3 | -21 + -3<br>-27 + -3 |

1-3- CONCLUSION DE L'ETUDE IN VIVO CHEZ LE SHR

Les produits de l'invention présentent une remarquable activité antihypertensive chez le rat SHR après administration de faibles doses par voie orale.
La durée d'action des produits décrits est considérablement allongée par rapport au meilleur produit de référence connu le CROMAKALIM pour une activité maximale comparable, et constitue un important avantage thérapeutique.

2- ETUDE CHEZ LE CHIEN EVEILLE HYPERTENDU RENAL

2-1- METHODOLOGIE

Des chiens bâtards de 20 à 25kg sont utilisés. Un cathéter en silastic mis en place sous anesthésie (pentobarbital sodique 30mg/kg IV) au niveau de l'aorte abdominale, laissé en chronique, permet la mesure ultérieure de la pression artérielle chez l'animal vigile.
L'hypertension artérielle est induite par une seconde intervention sous anesthésie consistant en une constriction de l'artère rénale gauche à l'aide d'un clip, réduisant son débit d'environ 70%, le rein gauche est enveloppé d'une capsule de latex, le rein controlatéral est laissé en place.
Les pressions artérielles systolique (PAS) et diastolique (PAD) sont mesurées par l'intermédiaire d'un capteur de pression STATHAM P23 connecté au cathéter en silastic et relié à un amplificateur de pression GOULD.
Les produits sont testés chez les animaux devenus hypertendus, à l'état éveillé, au moins une semaine après la seconde intervention. La pression artérielle est suivie en continu, sur un enregistreur GOULD ES1000 jusqu'à 7 heures après le traitement puis à 24 heures.
Les produits à tester sont administrés par la voie digestive après tubage gastrique sous forme dissoute dans l'eau ou le solvant approprié.
Les doses administrées sont exprimées en mg de base rapportée au poids corporel.

2-2- RESULTATS

Les produits représentatifs de l'invention ont été comparés au produit de référence ( CROMAKALIM ). Les variations des pressions artérielles systolique (PAS) et diastolique (PAD), présentées aux temps les plus significatifs après traitement, sont exprimées en pourcentage de variation par rapport aux valeurs contrôles.

| Produits testés | DOSE mg/kg | VALEURS CONTROLE mmHg | VARIATIONS ( %) EN FONCTION DU TEMPS APRES TRAITEMENT | | | | |
|---|---|---|---|---|---|---|---|
| | | | 30 mn | 1 H | 3 H | 7 H | 24 H |
| CROMAKALIM | 0.1 | PAS 189<br>PAD 93 | -50<br>-46 | -26<br>-23 | - 9<br>- 9 | + 2<br>- 5 | + 9<br>+18 |
| Exemple 1 forme B | 0.1 | PAS 199<br>PAD 99 | 0<br>0 | + 2<br>+ 5 | -55<br>-50 | -76<br>-68 | -24<br>-27 |
| Exemple 2 forme A | 0.1 | PAS 181<br>PAD 93 | - 2<br>-14 | -12<br>-20 | -28<br>-32 | -41<br>-44 | - 2<br>0 |

2-3- CONCLUSION DE L'ETUDE IN VIVO CHEZ LE CHIEN HYPERTENDU RENAL

La puissante activité antihypertensive et la longue durée d'action de ces produits est retrouvée chez le chien hypertendu rénal.
La supériorité du produit de l'exemple 1 forme B en terme de puissance et de durée d'action est notamment à souligner par rapport au CROMAKALIM.

3- ETUDE HEMODYNAMIQUE CHEZ LE CHIEN ANESTHESIE

3-1- METHODOLOGIE

Une étude hémodynamique complète a été réalisée sur des chiens bâtards de 25 à 30kg, anesthésiés au pentobarbital sodique 30mg/kg IV, intubés et ventilés, soumis à une thoracotomie au 5ème espace intercostal gauche.
La mesure des débits cardiaque et coronaire moyens est réalisée à l'aide de bagues électromagnétiques, mises en place au niveau de l'aorte ascendante et de la branche circonflexe de l'artère coronaire gauche respectivement, et reliées à des débitmètres électromagnétiques GOULD SP 2202. La pression aortique est mesurée par une sonde MILLAR introduite par voie artérielle fémorale.
Les valeurs de résistance vasculaire périphérique totale (TPR) et de résistance vasculaire coronaire moyenne (CVRM) sont calculées par le rapport de la pression aortique moyenne et du débit cardiaque moyen et du débit coronaire moyen respectivement.
Les produits testés ont été injectés par voie veineuse fémorale sous forme dissoute dans l'eau ou le solvant approprié.

3-2- RESULTATS

Les résultats obtenus avec les produits de l'invention testés sont comparés au produit de référence CROMAKALIM .
L'évolution des résistances vasculaires, exprimée en pourcentage de variation par rapport aux valeurs contrôles, est présentée en fonction du temps dans le tableau suivant.

| Produits testés | DOSE ug/kg | PARAMETRE | VARIATIONS ( %) EN FONCTION DU TEMPS APRES TRAITEMENT | | |
|---|---|---|---|---|---|
| | | | 1 mn | 10 mn | 30 mn |
| CROMAKALIM | 10 | CVRM<br>TPR | -69.0<br>-26.8 | -38.1<br>-15.5 | -27.4<br>-7.8 |
| Exemple 1<br>forme B | 10 | CVRM<br>TPR | -69.4<br>-45.9 | -62.0<br>-35.1 | -43.7<br>-35.1 |
| Exemple 1<br>forme A | 300 | CVRM<br>TPR | -81.5<br>-41.9 | -50.3<br>-17.2 | -34.9<br>-12.9 |
| Exemple 2<br>forme A | 30 | CVRM<br>TPR | -28.9<br>- 9.8 | -20.2<br>-11.8 | -22.1<br>-11.8 |
| Exemple 2<br>forme A | 100 | CVRM<br>TPR | -54.7<br>-15.8 | -56.1<br>-14.7 | -50.5<br>-12.6 |
| Exemple 3<br>forme A | 3000 | CVRM<br>TPR | + 2.5<br>+ 1.7 | -38.5<br>- 6.8 | -49.0<br>- 8.5 |

3-3- CONCLUSION DE L'ETUDE HEMODYNAMIQUE CHEZ LE CHIEN

Administrés par voie intraveineuse à faible dose, les composés de l'invention ont une activité vasodilatatrice périphérique et coronaire puissante et mieux soutenue que le CROMAKALIM .

B- D'une façon générale, l'ensemble des tests in vivo réalisés permet de conclure que les dérivés de la présente invention ont une activité comparable ou supérieure à celle du meilleur produit de référence connu ( CROMAKALIM ), tout en ayant une durée d'action beaucoup plus importante que ce dernier - ce qui constitue un atout majeur dans l'application thérapeutique.

**Exemple 22 :**

Etude pharmacologique dans le domaine bronchopulmonaire :

L'activité bronchodilatatrice a été étudiée, chez le cobaye par la méthode de H. KONZETT et R. ROSSLER, Arch. Exp. Path.

U. Pharm. 195, 71 (1940). On a ainsi observé que les dérivés de la présente invention, injectés par voie intraveineuse à des doses variant, selon les dérivés, de 0,3 à 1mg/kg inhibent notablement le bronchospasme provoqué par l'administration intraveineuse soit d'histamine, soit de sérotonine, soit d'acétylcholine.

Par exemple, le bronchospasme provoqué par l'administration IV d'histamine est inhibé de 96% avec le dérivé de l'exemple 1 forme B, à la dose de 0,30mg/kg IV et de 100% avec le dérivé de l'exemple 24 forme B, à la dose de 0,35mg/kg IV.

Soumis au test de A.K. ARMITAGE, Brit. J. Pharmacol., 17, 196 (1961), les dérivés testés, administrés par voie orale ou aérosol à des doses variant de 0,5 à 4mg/kg selon les composés, inhibent de 11 à 68% l'effet produit chez le cobaye par un aérosol d'histamine à 4%.

**Exemple 23** :

Preparation pharmaceutique :

Gélules dosées à 1mg de principe actif.

| | |
|---|---|
| 3R, 4S ou 3S, 4R trans diméthyl-2,2 hydroxy-3 (oxo-2 hydroxyméthyl-5S pyrrolidinyl-1)-4 cyano-6 chromane | 1mg |
| Amidon de maïs | 15mg |
| Lactose | 25mg |
| Talc | 5mg |

## Revendications

**Revendications pour les Etats contractants suivants : DE, GB, FR, IT, NL, SE, LI, CH, BE, AT, LU.**

**1.** Les dérivés de l'amino chromanol de formule générale I :

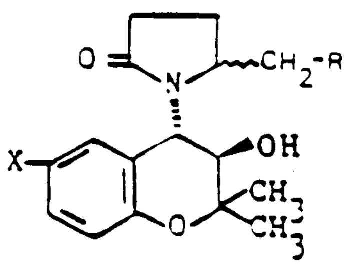

et

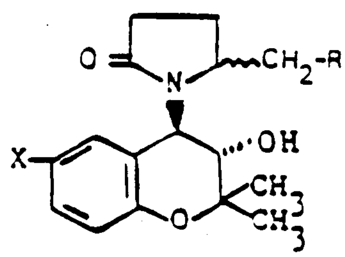

(I)

dans laquelle :

. X représente un radical cyano, nitro, trifluorométhyle, alkoxycarbonyle dans lequel le groupe alkoxy renferme de 1 à 5 atomes de carbone en chaîne droite ou ramifiée, un radical carbamido, dialkylcarbamido, aminosulfonyle, dialkylaminosulfonyle, alkylsulfonyle ou alkysulfonylamino dans lesquels le groupe alkyle renferme de 1 à 5 atomes de carbone, ou un radical acyle tel que par exemple un radical acétyle, propionyle, benzoyle ou trifluoroacétyle ;

. $CH_2$-R est fixé (en position 5 du noyau pyrrolidinone) sur un atome de carbone de configuration R ou S, et

. R représente :

a) soit un radical -OR' ou SR' dans lequel R' représente :

un atome d'hydrogène ;

un radical alkyle jusqu'en $C_{10}$, en chaîne droite ou ramifiée, comportant éventuellement un ou plusieurs atomes d'oxygène et/ou un radical hydroxy ;

un radical alkylcarbonyle dans lequel le groupe alkyle renferme jusqu'à 5 atomes de carbone en chaîne droite ou ramifiée ;

un reste

$$(CH_2)n-N<\begin{matrix}R_1\\R_2\end{matrix}$$

dans lequel

n est un nombre entier de 2 à 5

et $R_1$ , $R_2$ identiques ou différents représentent chacun :

. un atome d'hydrogène,

. un radical alkyle ayant jusqu'à 5 atomes de carbone en chaîne droite ou ramifiée, ou

. $R_1$ et $R_2$ forment ensemble avec l'atome d'azote auquel ils sont liés un cycle ayant 4 ou 5 atomes de carbone, comportant éventuellement un autre hétéroatome tel qu'un atome

d'oxygène ou de soufre, ou un groupe -N-$R_3$ dans lequel $R_3$ représente
- . un atome d'hydrogène,
- . un radical alkyle ayant jusqu'à 5 atomes de carbone en chaîne droite ou ramifiée, ou
- . un reste -AZ dans lequel A représente une liaison simple ou une chaîne hydrocarbonée de $C_1$ à $C_5$ comportant éventuellement une double liaison, et
  Z représente
  - . un radical hydroxy,
  - . un radical benzhydryle,
  - . un radical phényle éventuellement substitué par un ou plusieurs atomes d'halogène, ou radicaux trifluorométhyle, alkoxy de $C_1$ à $C_5$ ou méthylènedioxy, ou
  - . un radical hétérocyclique penta ou hexagonal comportant un ou plusieurs atomes d'oxygène, d'azote ou de soufre tels que les radicaux pyridyle, thiazolyle, pyrimidinyle, ou pyrazinyle, chacun éventuellement substitué par un ou plusieurs atomes d'halogène ou radicaux alkyle ou alkoxy chacun de $C_1$ à $C_5$ ;

b) soit un radical

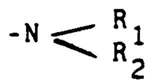

dans lequel $R_1$ et $R_2$ ont les significations énoncées précédemment.

**2.** Les sels des dérivés de la revendication 1 répondant à la formule générale I dans le cas où R contient une fonction basique, avec des acides appropriés.

**3.** Les sels selon la revendication 2 qui sont physiologiquement tolérables.

**4.** Le 3R, 4S ou 3S, 4R trans diméthyl-2,2 hydroxy-3 (oxo-2 hydroxyméthyl-5S pyrrolidinyl-1)-4 cyano-6 chromane, sous forme A et B.

**5.** Le 3S, 4R ou 3R, 4S trans diméthyl-2,2 hydroxy-3 (oxo-2 méthoxyméthyl-5S pyrrolidinyl-1)-4 cyano-6 chromane, sous forme A et B.

**6.** Le 3R, 4S ou 3S, 4R trans diméthyl-2,2 hydroxy-3 (oxo-2 diméthylaminométhyl-5S pyrrolidinyl-1)-4 cyano-6 chromane, sous forme A et B.

**7.** Le 3R, 4S, ou 3S, 4R trans diméthyl-2,2 hydroxy-3 (oxo-2 hydroxyméthyl-5S pyrrolidinyl-1)-4 nitro-6 chromane, sous forme A et B.

**8.** Le 3R, 4S ou 3S, 4R trans diméthyl-2,2 hydroxy-3 (oxo-2 hydroxyméthyl-5S pyrrolidinyl-1)-4 méthoxycarbonyl-6 chromane, sous forme A et B.

**9.** Le procédé de préparation des dérivés de la revendication 1 caractérisé en ce que l'on fait réagir en quantité équimoléculaire le dérivé du chromane de formule générale II :

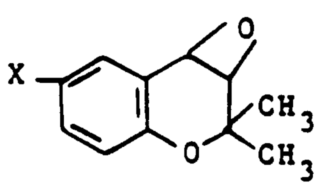

(II)

dans laquelle X a la signification définie dans la revendication 1,
sur le dérivé chiral de configuration R ou S de la pyrrolidone de formule générale III :

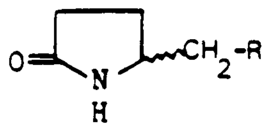

(III)

dans laquelle R a la signification définie dans la revendication 1, à l'état de sel de sodium ou de lithium.

**10.** Le procédé de préparation selon la revendication 9 caractérisé en ce que la réaction est effectuée dans un solvant aprotique à une température comprise entre 20 et 50 ° C.

**11.** Les compositions pharmaceutiques contenant comme principe actif un composé selon les revendications 1 et 3 à 8, avec des excipients pharmaceutiques appropriés.

**12.** Les compositions pharmaceutiques selon la revendication 11, présentées sous une forme convenant notamment pour le traitement des troubles de régulation de la contraction du muscle lisse dans les sphères cardiovasculaire, bronchopulmonaire, digestive, urinaire et utérine, des pathologies ischémiques, inflammatoires, oedemateuses ou prolifératives au niveau organique ou cutané, et des maladies métaboliques avec obésité et trouble de glycorégulation.

**Revendications pour les Etats contractants suivants: ES, GR.**

**1.** Le procédé de préparation des dérivés de l'amino chromanol de formule générale I :

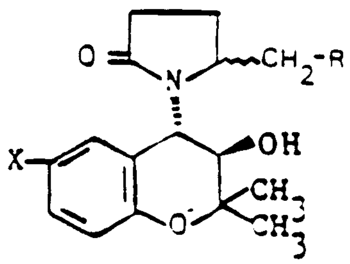

et

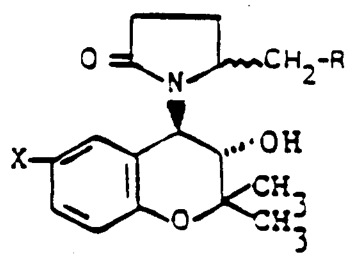

(I)

dans laquelle :

. X représente un radical cyano, nitro, trifluorométhyle, alkoxycarbonyle dans lequel le groupe alkoxy renferme de 1 à 5 atomes de carbone en chaîne droite ou ramifiée, un radical carbamido, dialkylcarbamido, aminosulfonyle, dialkylaminosulfonyle, alkylsulfonyle ou alkysulfonylamino dans lesquels le groupe alkyle renferme de 1 à 5 atomes de carbone, ou un radical acyle tel que par exemple un radical acétyle, propionyle, benzoyle ou trifluoroacétyle ;

. $CH_2$-R est fixé (en position 5 du noyau pyrrolidinone) sur un atome de carbone de configuration R ou S, et

. R représente :

a) soit un radical -OR' ou SR' dans lequel R' représente :

un atome d'hydrogène ;

un radical alkyle jusqu'en $C_{10}$, en chaîne droite ou ramifiée, comportant éventuellement un ou plusieurs atomes d'oxygène et/ou un radical hydroxy ;

un radical alkylcarbonyle dans lequel le groupe alkyle renferme jusqu'à 5 atomes de carbone en chaîne droite ou ramifiée ;

un reste

$$(CH_2)n-N<{}^{R_1}_{R_2}$$

dans lequel

n est un nombre entier de 2 à 5

et $R_1$ , $R_2$ identiques ou différents représentent chacun :

- . un atome d'hydrogène,
- . un radical alkyle ayant jusqu'à 5 atomes de carbone en chaîne droite ou ramifiée, ou
- . $R_1$ et $R_2$ forment ensemble avec l'atome d'azote auquel ils sont liés un cycle ayant 4 ou 5 atomes de carbone, comportant éventuellement un autre hétéroatome tel qu'un atome d'oxygène ou de soufre, ou un groupe -N-$R_3$ dans lequel $R_3$ représente
- . un atome d'hydrogène,
- . un radical alkyle ayant jusqu'à 5 atomes de carbone en chaîne droite ou ramifiée, ou
- . un reste -AZ dans lequel A représente une liaison simple ou une chaîne hydrocarbonée de $C_1$ à $C_5$ comportant éventuellement une double liaison, et

  Z représente

  - . un radical hydroxy,
  - . un radical benzhydryle,
  - . un radical phényle éventuellement substitué par un ou plusieurs atomes d'halogène, ou radicaux trifluorométhyle, alkoxy de $C_1$ à $C_5$ ou méthylènedioxy, ou
  - . un radical hétérocyclique penta ou hexagonal comportant un ou plusieurs atomes d'oxygène, d'azote ou de soufre tels que les radicaux pyridyle, thiazolyle, pyrimidinyle, ou pyrazinyle, chacun éventuellement substitué par un ou plusieurs atomes d'halogène ou radicaux alkyle ou alkoxy chacun de $C_1$ à $C_5$ ;

b) soit un radical

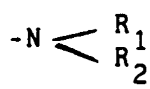

dans lequel $R_1$ et $R_2$ ont les significations énoncées précédemment ;

et de leurs sels, dans le cas où R contient une fonction basique, avec des acides appropriés ;

caractérisé en ce que l'on fait réagir en quantité équimoléculaire le dérivé du chromane de formule générale II :

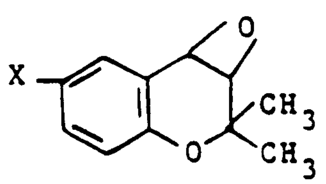

(II)

dans laquelle X a la signification précédemment définie, sur le dérivé chiral de configuration R ou S de la pyrrolidone de formule générale III :

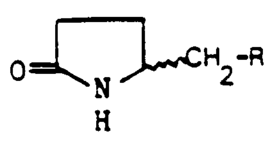

(III)

dans laquelle R a la signification précédemment définie à l'état de sel de sodium ou de lithium.

**2.** Le procédé de préparation selon la revendication 1 caractérisé en ce que la réaction est effectuée dans un solvant aprotique à une température comprise entre 20 et 50 ° C.

**Claims**

**Claims for the following Contracting States : DE, GB, FR, IT, NL, SE, LI, CH, BE, AT, LU**

**1.** Aminochromanol compounds of the general formula I:

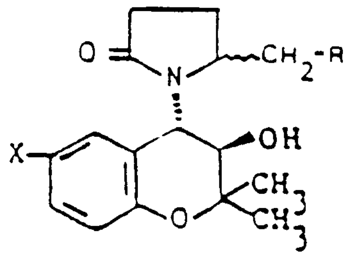

and

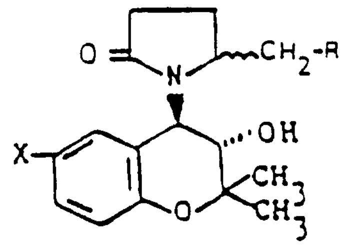

(I)

in which:

- X represents a cyano radical, a nitro radical, a trifluoromethyl radical, an alkoxycarbonyl radical in which the alkoxy group contains from 1 to 5 carbon atoms in a straight or branched chain, a carbamoyl radical, a dialkylcarbamoyl radical, an aminosulphonyl radical, a dialkylaminosulphonyl radical, an alkylsulphonyl radical or an alkylsulphonylamino radical in each of which an alkyl group contains from 1 to 5 carbon atoms, or an acyl radical, such as, for example, an acetyl, propionyl, benzoyl or trifluoroacetyl radical;
- $CH_2$-R is attached (in the 5-position of the pyrrolidinone nucleus) to a carbon atom and has the R or S configuration, and
- R represents:

  a) either a radical -OR' or SR' in which R' represents:

  a hydrogen atom;

  an alkyl radical containing up to 10 carbon atoms in a straight or branched chain and optionally containing one or more oxygen atoms and/or a hydroxy radical;

  an alkylcarbonyl radical in which the alkyl group contains up to 5 carbon atoms in a straight or branched chain;

  a radical

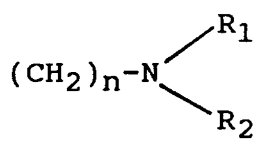

  in which

  n is an integer from 2 to 5

  and each of $R_1$ and $R_2$, which are identical or different, represents:
  - a hydrogen atom or
  - an alkyl radical having up to 5 carbon atoms in a straight or branched chain, or

  $R_1$ and $R_2$, together with the nitrogen atom to which they are attached, form a ring that has 4 or 5 carbon atoms and that optionally contains another hetero atom, such as an oxygen atom or a sulphur atom, or a group -N-$R_3$ in which $R_3$ represents:
  - a hydrogen atom,
  - an alkyl radical having up to 5 carbon atoms in a straight or branched chain, or
  - a radical -AZ in which A represents a single bond or a $C_1$-$C_5$ hydrocarbon chain optionally containing a double bond, and Z represents:
    - a hydroxy radical,
    - a benzhydryl radical,
    - a phenyl radical optionally substituted by one or more halogen atoms or trifluoromethyl, $C_1$-$C_5$-alkoxy or methylenedioxy radicals, or
    - a pentagonal or hexagonal heterocyclic radical containing one or more oxygen, nitrogen or sulphur atoms, such as the pyridyl, thiazolyl, pyrimidinyl or pyrazinyl radicals, each optionally substituted by one or more halogen atoms or $C_1$-$C_5$-alkyl or $C_1$-$C_5$-alkoxy

radicals;

b) or a radical

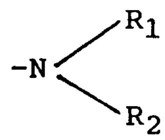

in which $R_1$ and $R_2$ have the meanings given above.

**2.** The salts of the compounds of claim 1 corresponding to the general formula I, when R contains a basic function, with suitable acids.

**3.** The salts according to claim 2 which are physiologically tolerable.

**4.** 3R,4S- or 3S,4R-trans-2,2-dimethyl-3-hydroxy-4-(2-oxo-5S-hydroxymethyl-1-pyrrolidinyl)-6-cyanochroman, in the A and B form.

**5.** 3S,4R- or 3R,4S-trans-2,2-dimethyl-3-hydroxy-4-(2-oxo-5S-methoxymethyl-1-pyrrolidinyl)-6-cyanochroman, in the A and B form.

**6.** 3R,4S- or 3S,4R-trans-2,2-dimethyl-3-hydroxy-4-(2-oxo-5S-dimethylaminomethyl-1-pyrrolidinyl)-6-cyanochroman, in the A and B form.

**7.** 3R,4S- or 3S,4R-trans-2,2-dimethyl-3-hydroxy-4-(2-oxo-5S-hydroxymethyl-1-pyrrolidinyl)-6-nitrochroman, in the A and B form.

**8.** 3R,4S- or 3S,4R-trans-2,2-dimethyl-3-hydroxy-4-(2-oxo-5S-hydroxymethyl-1-pyrrolidinyl)-6-methoxycarbonylchroman, in the A and B form.

**9.** Process for the preparation of the compounds of claim 1, characterised in that the chroman compound of the general formula II:

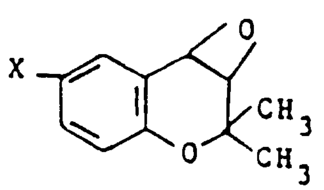

(II),

in which X has the meaning defined in claim 1, is reacted, in an equimolar amount, with the chiral pyrrolidone compound, having the R or S configuration, of the general formula III:

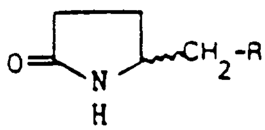

(III),

in which R has the meaning defined in claim 1, in the form of the sodium or lithium salt.

**10.** Preparation process according to claim 9, characterised in that the reaction is carried out in an aprotic solvent at a temperature of from 20 to 50 °C.

**11.** Pharmaceutical compositions containing as active ingredient a compound according to any one of claims 1 and 3 to 8, with suitable pharmaceutical excipients.

**12.** Pharmaceutical compositions according to claim 11 in a form suitable especially for the treatment of disorders in the regulation of the contraction of smooth muscle in the cardiovascular, bronchopulmonary, digestive, urinary and uterine fields, of ischaemic, inflammatory, oedematous or proliferative pathologies at the organ or cutaneous level, and of metabolic diseases associated with obesity and glycoregulation disorders.

**Claims for the following Contracting States : ES, GR**

**1.** Process for the preparation of aminochromanol compounds of the general formula I: in

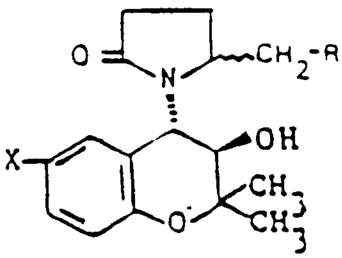 and 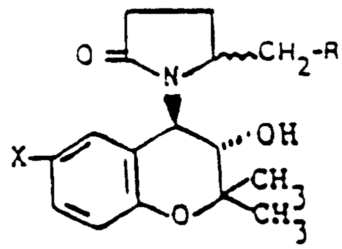 (I)

which:

- X represents a cyano radical, a nitro radical, a trifluoromethyl radical, an alkoxycarbonyl radical in which the alkoxy group contains from 1 to 5 carbon atoms in a straight or branched chain, a carbamoyl radical, a dialkylcarbamoyl radical, an aminosulphonyl radical, a dialkylaminosulphonyl radical, an alkylsulphonyl radical or an alkylsulphonylamino radical in each of which an alkyl group contains from 1 to 5 carbon atoms, or an acyl radical, such as, for example, an acetyl, propionyl, benzoyl or trifluoroacetyl radical;
- $CH_2$-R is attached (in the 5-position of the pyrrolidinone nucleus) to a carbon atom and has the R or S configuration, and
- R represents:
  a) either a radical -OR' or SR' in which R' represents:
  a hydrogen atom;
  an alkyl radical containing up to 10 carbon atoms in a straight or branched chain and optionally containing one or more oxygen atoms and/or a hydroxy radical;
  an alkylcarbonyl radical in which the alkyl group contains up to 5 carbon atoms in a straight or branched chain;
  a radical

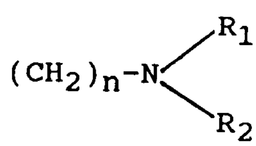

  in which
  n is an integer from 2 to 5
  and each of $R_1$ and $R_2$, which are identical or different, represents:
  - a hydrogen atom or
  - an alkyl radical having up to 5 carbon atoms in a straight or branched chain, or

  $R_1$ and $R_2$, together with the nitrogen atom to which they are attached, form a ring that has 4 or 5 carbon atoms and that optionally contains another hetero atom, such as an oxygen atom or a sulphur atom, or a group -N-$R_3$ in which $R_3$ represents:
  - a hydrogen atom,
  - an alkyl radical having up to 5 carbon atoms in a straight or branched chain, or
  - a radical -AZ in which
    A represents a single bond or a $C_1$-$C_5$ hydrocarbon chain optionally containing a double bond, and
    Z represents:

- a hydroxy radical,
- a benzhydryl radical,
- a phenyl radical optionally substituted by one or more halogen atoms or trifluoromethyl, $C_1$-$C_5$-alkoxy or methylenedioxy radicals, or
- a pentagonal or hexagonal heterocyclic radical containing one or more oxygen, nitrogen or sulphur atoms, such as the pyridyl, thiazolyl, pyrimidinyl or pyrazinyl radicals, each optionally substituted by one or more halogen atoms or $C_1$-$C_5$-alkyl or $C_1$-$C_5$-alkoxy radicals;

b) or a radical

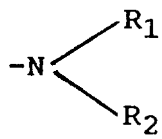

in which $R_1$ and $R_2$ have the meanings given above;
and their salts, when R contains a basic function, with suitable acids, characterised in that the chroman compound of the general formula II:

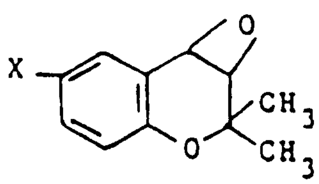

(II),

in which X has the meaning defined above,
is reacted, in an equimolar amount, with the chiral pyrrolidone compound, having the R or S configuration, of the general formula III:

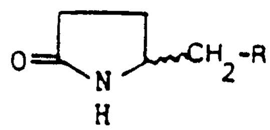

(III),

in which R has the meaning defined above,
in the form of the sodium or lithium salt.

2. Preparation process according to claim 1, characterised in that the reaction is carried out in an aprotic solvent at a temperature of from 20 to 50 °C.

**Patentansprüche**
**Patentansprüche für folgende Vertragsstaaten : DE, GB, FR, IT, NL, SE, LI, CH, BE, AT, LU**

1. Aminochromanol-Derivate der allgemeinen Formel I:

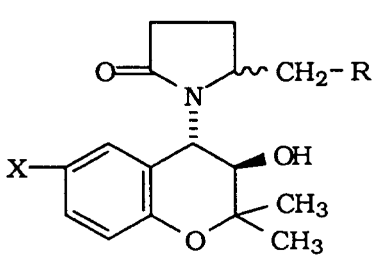

und

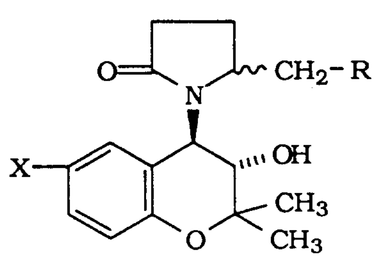

(I)

worin

. X eine Cyanogruppe, eine Nitrogruppe, eine Trifluormethylgruppe, eine Alkoxycarbonylgruppe, worin die Alkoxygruppe 1 bis 5 Kohlenstoffatome in gerader oder verzweigter Kette enthält, eine Carbamidogruppe, eine Dialkylcarbamidogruppe, eine Aminosulfonylgruppe, eine Dlalkylaminosulfonylgruppe, eine Alkylsulfonylgruppe oder eine Alkylsulfonylaminogruppe, worin die Alkylgruppe 1 bis 5 Kohlenstoffatome enthält, oder eine Acylgruppe, wie beispielsweise eine Acetylgruppe, eine Propenylgruppe, eine Benzoylgruppe oder eine Trifluoracetylgruppe;

. $CH_2$-R (in der 5-Stellung des Pyrrolidinonrings) an ein Kohlenstoffatom in der Konfiguration R oder S gebunden ist, und

. R

a) entweder eine Gruppe -OR' oder -SR', worin R' ein Wasserstoffatom;

eine geradkettige oder verzweigte Alkylgruppe bis $C_{10}$, die gegebenenfalls eines oder mehrere Sauerstoffatome und/oder eine Hydroxylgruppe aufweist;

eine Alkylcarbonylgruppe, worin die Alkylgruppe bis zu 5 Kohlenstoffatome in gerader oder verzweigter Kette enthält;

einen Rest der Formel

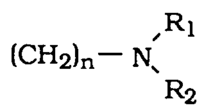

worin n eine ganze Zahl mit einem Wert von 2 bis 5 und

$R_1$ und $R_2$, die gleichartig oder verschieden sein können, jeweils

. Wasserstoffatome,

. eine Alkylgruppe mit bis zu 5 Kohlenstoffatomen in gerader oder verzweigter Kette bedeuten, oder

. $R_1$ und $R_2$ gemeinsam mit dem Stickstoffatom, an das sie gebunden sind, einen Ring mit 4 oder 5 Kohlenstoffatomen bilden, der gegebenenfalls ein weiteres Heteroatom, wie ein Sauerstoffatom oder ein Schwefelatom, oder eine Gruppe -N-$R_3$ aufweist, worin $R_3$

. ein Wasserstoffatom,

. eine Alkylgruppe mit bis zu 5 Kohlenstoffatomen in gerader oder verzweigter Kette oder

. einen Rest -AZ darstellt, worin

A eine Einfachbindung oder eine $C_1$-$C_5$-Kohlenwasserstoffkette, die gegebenenfalls eine Doppelbindung aufweist und

Z

. eine Hydroxylgruppe,

. eine Benzhydrylgruppe,

. eine Phenylgruppe, die gegebenenfalls durch ein oder mehrere Halogenatome, oder Trifluormethylgruppen, $C_1$-$C_5$-Alkoxygruppen oder Methylendioxygruppen substituiert ist, oder

. eine pentagonale oder hexagonale heterocyclische Gruppe, die eines oder mehrere Sauerstoffatome, Stickstoffatome oder Schwefelatome aufweist, wie eine Pyridylrest, einen Thiazolylrest, einen Pyrimidinylrest oder einen Pyrazinylrest, die jeweils gegebenenfalls durch ein oder mehrere Halogenatome, $C_1$-$C_5$-Alkylgruppen oder $C_1$-$C_5$-Alkoxygruppen substituiert sind, bedeuten, darstellt; oder

b) eine Gruppe der Formel

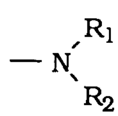

worin $R_1$ und $R_2$ die oben angegebenen Bedeutungen besitzen, bedeuten.

**2.** Die Salze der Derivate nach Anspruch 1 der allgemeinen Formel I, worin R eine basische Funktion aufweist, mit geeigneten Säuren.

**3.** Die Salze nach Anspruch 2, die physiologisch verträglich sind.

**4.** 3R,4S- oder 3S,4R-trans-2,2-Dimethyl-3-hydroxy-4-(2-oxo-5S-hydroxymethyl-pyrrolidin-1-yl)-6-cyano-chroman in der Form A und B.

**5.** 3S,4R- oder 3R,4S-trans-2,2-Dimethyl-3-hydroxy-4-(2-oxo-5S-methoxymethyl-pyrrolidin-1-yl)-6-cyano-chroman in der Form A und B.

**6.** 3R,4S- oder 3S,4R-trans-2,2-Dimethyl-3-hydroxy-4-(2-oxo-5S-dimethylaminomethyl-pyrrolidin-1-yl)-6-cyano-chroman in der Form A und B.

**7.** 3R,4S- oder 3S,4R-trans-2,2-Dimethyl-3-hydroxy-4-(2-oxo-5S-hydroxymethyl-pyrrolidin-1-yl)-6-nitro-chroman in der Form A und B.

**8.** 3R,4S- oder 3S,4R-trans-2,2-Dimethyl-3-hydroxy-4-(2-oxo-5S-hydroxymethyl-pyrrolidin-1-yl)-6-methoxycarbonyl-chroman in der Form A und B.

**9.** Verfahren zur Herstellung der Derivate nach Anspruch 1, **dadurch gekennzeichnet**, daß man das Chromanderivat der allgemeinen Formel II:

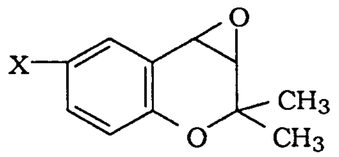

(II)

in der X die in Anspruch 1 angegebenen Bedeutungen besitzt, in äquimolarer Menge mit dem chiralen Derivat der Konfiguration R oder S des Pyrrolidinons der allgemeinen Formel III:

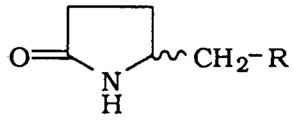

(III)

in der R die in Anspruch 1 angegebenen Bedeutungen besitzt, in Form des Natriumsalzes oder des Lithiumsalzes umsetzt.

**10.** Verfahren nach Anspruch 9, **dadurch gekennzeichnet**, daß die Reaktion in einem aprotischen Lösungsmittel bei einer Temperatur zwischen 20 und 50 °C durchgeführt wird.

**11.** Pharmazeutische Zubereitungen enthaltend als Wirkstoff eine Verbindung nach einem der Ansprüche 1 und 3 bis 8 zusammen mit geeigneten pharmazeutischen Trägermaterialien.

**12.** Pharmazeutische Zubereitungen nach Anspruch 11 in einer Form, die insbesondere zur Behandlung von Störungen der Steuerung der Kontraktion von glatten Muskeln im kardiovaskulären, bronchopulmonalen Bereich, Verdauungsbereich, urinären Bereich und Uterusbereich, von ischämischen, inflammatorischen, ödematösen oder proliferativen pathologischen Zuständen im Organbereich oder Hautbereich und von Stoffwechselerkrankungen mit Fettsucht und Zuckerregulierungsstörungen.

**Patentansprüche für folgende Vertragsstaaten : ES, GR**

1. Verfahren zur Herstellung von Aminochromanol-Derivaten der allgemeinen Formel I:

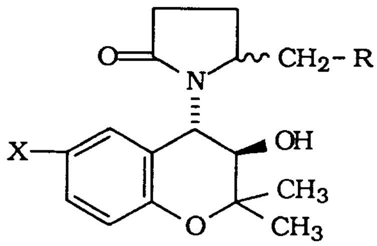

und

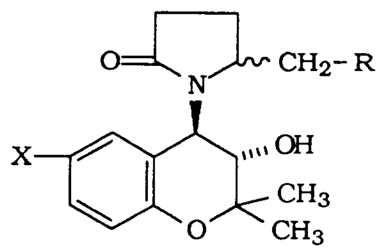

(I)

worin

. X eine Cyanogruppe, eine Nitrogruppe, eine Trifluormethylgruppe, eine Alkoxycarbonylgruppe, worin die Alkoxygruppe 1 bis 5 Kohlenstoffatome in gerader oder verzweigter Kette enthält, eine Carbamidogruppe, eine Dialkylcarbamidogruppe, eine Aminosulfonylgruppe, eine Dialkylaminosulfonylgruppe, eine Alkylsulfonylgruppe oder eine Alkylsulfonylaminogruppe, worin die Alkylgruppe 1 bis 5 Kohlenstoffatome enthält, oder eine Acylgruppe, wie beispielsweise eine Acetylgruppe, eine Propenylgruppe, eine Benzoylgruppe oder eine Trifluoracetylgruppe;

. $CH_2$-R (in der 5-Stellung des Pyrrolidinonrings) an ein Kohlenstoffatom in der Konfiguration R oder S gebunden ist, und

. R

a) entweder eine Gruppe -OR' oder -SR', worin R' ein Wasserstoffatom;

eine geradkettige oder verzweigte Alkylgruppe bis $C_{10}$, die gegebenenfalls eines oder mehrere Sauerstoffatome und/oder eine Hydroxylgruppe aufweist;

eine Alkylcarbonylgruppe, worin die Alkylgruppe bis zu 5 Kohlenstoffatome in gerader oder verzweigter Kette enthält;

einen Rest der Formel

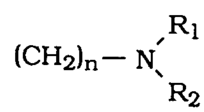

worin n eine ganze Zahl mit einem Wert von 2 bis 5 und $R_1$ und $R_2$, die gleichartig oder verschieden sein können, jeweils

. Wasserstoffatome,

. eine Alkylgruppe mit bis zu 5 Kohlenstoffatomen in gerader oder verzweigter Kette bedeuten, oder

. $R_1$ und $R_2$ gemeinsam mit dem Stickstoffatom, an das sie gebunden sind, einen Ring mit 4 oder 5 Kohlenstoffatomen bilden, der gegebenenfalls ein weiteres Heteroatom, wie ein Sauerstoffatom oder ein Schwefelatom, oder eine Gruppe -N-$R_3$ aufweist, worin $R_3$

. ein Wasserstoffatom,

. eine Alkylgruppe mit bis zu 5 Kohlenstoffatomen in gerader oder verzweigter Kette oder

. einen Rest -AZ darstellt, worin A eine Einfachbindung oder eine $C_1$-$C_5$-Kohlenwasserstoffkette, die gegebenenfalls eine Doppelbindung aufweist und

Z

. eine Hydroxylgruppe,

. eine Benzhydrylgruppe,

. eine Phenylgruppe, die gegebenenfalls durch ein oder mehrere Halogenatome, oder Trifluormethylgruppen, $C_1$-$C_5$-Alkoxygruppen oder Methylendioxygruppen substituiert ist, oder

. eine pentagonale oder hexagonale heterocyclische Gruppe, die eines oder mehrere Sauerstoffatome, Stickstoffatome oder Schwefelatome aufweist, wie eine Pyridylrest,

einen Thiazolylrest, einen Pyrimidinylrest oder einen Pyrazinylrest, die jeweils gegebenenfalls durch ein oder mehrere Halogenatome, $C_1$-$C_5$-Alkylgruppen oder $C_1$-$C_5$-Alkoxygruppen substituiert sind, bedeuten, darstellt; oder

b) eine Gruppe der Formel

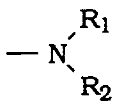

worin $R_1$ und $R_2$ die oben angegebenen Bedeutungen besitzen, bedeuten; sowie deren Salze dann, wenn Reine basische Funktion aufweist, mit geeigneten Säuren, **dadurch gekennzeichnet,** daß man das Chromanderivat der allgemeinen Formel II:

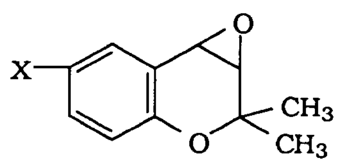

(II)

in der X die in Anspruch 1 angegebenen Bedeutungen besitzt, in äquimolarer Menge mit dem chiralen Derivat der Konfiguration R oder S dersPyrrolidinons der allgemeinen Formel III:

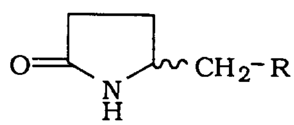

(III)

in der R die in Anspruch 1 angegebenen Bedeutungen besitzt, in Form des Natriumsalzes oder des Lithiumsalzes umsetzt.

2. Verfahren nach Anspruch 1, **dadurch gekennzeichnet**, daß die Reaktion in einem aprotischen Lösungsmittel bei einer Temperatur zwischen 20 und 50 °C durchgeführt wird.